# EUROPEAN PATENT APPLICATION

(11) **EP 1 249 785 A2**
(43) Date of publication of application: **16.10.2002**
(21) Application number: 01128950.1
(22) Date of filing: 06.12.2001
(51) Int. Cl.: G06F 19/00

(54) **Method and system to report on occupational health facility**

(30) Priority: 14.12.2000 GB 0030548
(71) Applicant: CIMO SA, 1870 Monthey (CH)
(72) Inventor: Turin, Raymond, c/o CIMO S.A., 1870 Monthey (CH); Bellagamba, Jean-Marc, c/o CIMO S.A., 1870 Monthey (CH)
(74) Representative: Bastian, Werner Maria

(57) **Abstract**

There is provided an information reporting system for an occupational health facility for use with an electronic database, said electronic database containing records for a group of individuals, there being at least one record for each individual, said records containing historical information on the work place exposure, generated by a first database application means from at least three independent files, said files representing the work place data, the product risk data and the personnel data, said reporting system further comprising:
a second database application means for incorporating at least legislative requirement data into said records for each individual; and
a report generating means for generating
   a) a schedule for operating the occupational health facility, and/or
   b) an epidemiological analysis on the total or part of the individual records.

## Description

The invention relates to an information reporting system for an occupational health facility, a method of reporting said clinical information and a data processing system to carry out said method.

The advances in technology and particularly the synthesis of new chemical compounds require a better knowledge and understanding in the area of occupational health. At the present time, it is almost impossible to evaluate in a definite way the influence of the exposure to new technologies or to new chemical compounds. It is therefore necessary to be able to re-evaluate the situation, especially with regard to past exposure, according to the development of further knowledge. Unfortunately, up to now a re-evaluation of an initial assessment as to the risks associated with the exposure to a technology or to a chemical compound is hardly ever undertaken. In any event such re-evaluation is up to now only possible by carrying out one change at a time, whereby neglecting possible interactions of exposures. In addition to this limitation, the ever increasing mobility of employees between different work places and legislative changes imposing additional medical exams or check-ups are to be taken into account in developing an information reporting system for an occupational health facility.

It is therefore an object of the present invention to provide an information reporting system for an occupational health facility, which generates a schedule for operating the occupational health facility.

It is a further object of the present invention to provide an information reporting system for an occupational health facility, which generates an epidemiological analysis on the total or part of the individual records.

In contrast to the existing proposals, according to a first aspect of the present invention, there is provided an information reporting system for an occupational health facility for use with an electronic database, said electronic database containing records for a group of individuals, there being at least one record for each individual, said records containing historical information on the work place exposure, generated by a first database application means from at least three independent files, said files representing the work place data, the product risk data and the personnel data, said reporting system further comprising:
a second database application means for incorporating at least legislative requirement data into said records for each individual; and
a report generating means for generating
   a) a schedule for operating the occupational health facility, and/or
   b) an epidemiological analysis on the total or part of the individual records.

An advantage of the information reporting system according to the present invention is the accessibility of the data and the possibility to perform almost instantly an analysis of the data.

A further advantage of the information reporting system according to the present invention is the possibility to export the data generated in the course of an epidemiological analysis for further use.

A further advantage of the information reporting system according to the present invention is the possibility to track an individual's exposure over a whole working life.

A further advantage of the information reporting system according to the present invention is the possibility to retroactively track personnel where a potential health risk is only later discovered.

A further advantage of the information reporting system according to the present invention is the possibility for the user to define his/her own parameters with which the system is operated. These parameters include the medical parameters or criteria as well as the parameters of exposure.

A further advantage of the information reporting system according to the present invention is the possibility to use pre-defined, standardized lists of values, so that the user of the system is guided when data is put into the system. Not only errors will be avoided in doing so but also the later analysis of the data will be facilitated.

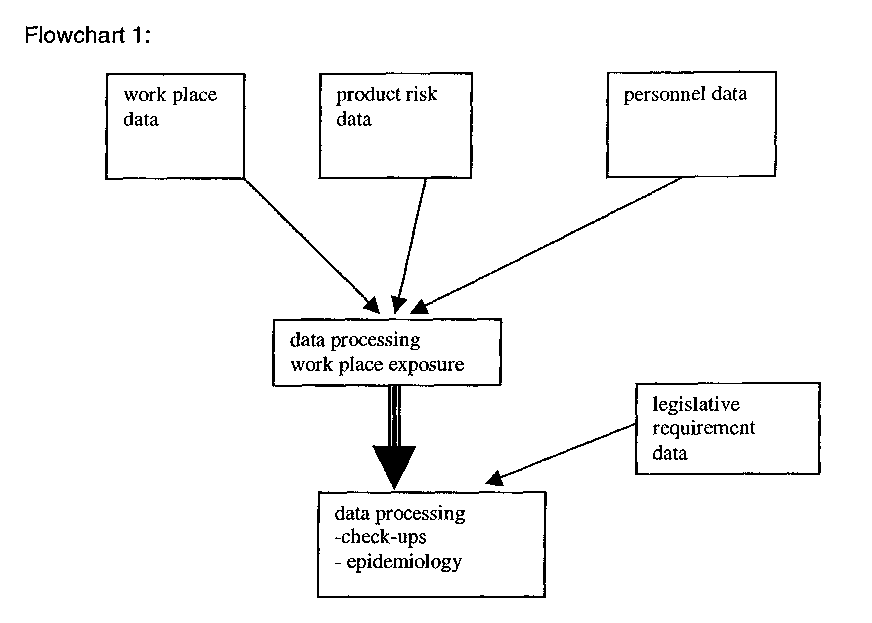

Above flowchart (1) is a schematic representation of the principal elements of the information reporting system. At least three independent files representing the work place data, the product risk data and the personnel data are used to define the work place exposure of each individual which is then used for generating the reports after taking into consideration the legislative requirements represented by the legislative requirement data.

The work place data file contains information about a particular work place and its changes over time. The data is characteristic for each work place at a given point in time and usually requires a work place analysis. Said work place analysis will result, for example, in information on the exposure to chemical compounds or on the exposure related to certain techniques like computer screens. Another possibility is to rely on work place information, which is available for a standardized work place. Such standardized work place information is increasingly available so to avoid a repetitive work place analysis. Said work place analysis comprises any kind of potential danger such as biological, physical, chemical or psychological dangers (for example vibrations, exposure to chemicals, etc.).

The product risk data file contains information about a risk associated with a particular product whereby product comprises not only chemical compounds but also the working environment in general. Said data is also susceptible to change over time as a function of the further development of knowledge. Said product risk information may be obtained from external sources or generated in-house.

The personnel data file contains information about the individuals, such as name, age, gender and address; usually these individuals are the employees of a given company or of associated firms. Said data also includes the different work places that said individual has occupied over time. Preferably said data is imported directly from a company's central personnel database each time a change has occurred.

In addition to the work place data file, the product risk data file and the personnel data file, there may be used other data files comprising, for example, general health conditions that might influence the work place exposure determination.

The legislative requirement data relate to limits of exposure as well as to regulations imposing certain medical exams or check-up. The regulations for medical exams may vary considerably depending on the exposure or country, for example, and are also susceptible to be revised from time to time.

In addition to the strictly legislative requirement data, there may be a number of internally set limits on certain exposures or additional exams or also suggested vaccinations, which in addition to the legislative requirements determine the schedule for operating the occupational health facility. Such additional factors can be taken into consideration in the second database application.

In addition the information reporting system for an occupational health facility may include a further data base application means for incorporating diagnosis data into the records for each individual. Said diagnosis data may comprise data from biological analysis, such as blood or urine, for example, but also on treatments and prescriptions. By way of a standardized diagnosis language, this data is also very useful in carrying out epidemiological analysis.

In addition the information reporting system for an occupational health facility may include one or more lists of pre-defined, standardized values for certain data. Such lists of values help the user of the system when data is put into the system.

In a further aspect the present invention relates to a method of establishing the work place exposure of an individual using a database application means for generating said work place exposure from at least three independent files, said files representing the work place data, the product risk data and the personnel data. The work place exposure data of an individual generated by above method may then be used for further analysis or calculations.

In a further aspect the present invention relates to a method of reporting information about a group of individuals comprising
storing records for the group of individuals, there being at least one record for each individual containing the historical information on the work place exposure of each individual generated from at least three independent files, said files representing the work place data, the product risk data and the personnel data,
storing legislative requirement data,
incorporating at least said legislative requirement data into said records for each individual,
generating a schedule for operating an occupational health facility and/or
generating an epidemiological analysis on the total or part of the individual records.

In a further aspect the present invention relates to a data processing system for carrying out above method of reporting data comprising
input means for collecting the different data needed,
storage means for at least three independent files representing the work place data, the product risk data and the personnel data,
a first database application means for generating the historical information on the work place exposure of each individual from at least three independent files, said files representing the work place data, the product risk data and the personnel data,
storage means for at least a file representing legislative requirement data,
a second database application means for incorporating at least legislative requirement data into said records for each individual;
a report generating means for generating
a schedule for operating the occupational health facility, and/or
an epidemiological analysis on the total or part of the individual records; and
output means for the presentation of said reports.

As input means, it is possible to use a conventional keyboard, for example. It is also possible to input data through an electronic transfer of data, like importing a file formatted in a defined way.

As storage means, it is possible to use any conventional storage means, for example a magnetic disk or tape or so-called memory sticks.

As output means, it is possible to use a conventional printer or on-screen output means, for example. It is also possible to output data through an electronic transfer of data, like exporting a file formatted in a defined way.

In a more general way, the information reporting system can also be integrated into a standard office environment comprising text editors (for example MS Word), spreadsheet calculating programs (for example MS Excel) or mail programs (for example MS Outlook). Such integration facilitates data exchange including data input and data output.

Preferably the data processing system further comprises at least one workstation, one host computer and a network interconnecting said host computer and workstation for enabling communication there between.

## Claims

1. An information reporting system for an occupational health facility for use with an electronic database, said electronic database containing records for a group of individuals, there being at least one record for each individual, said records containing historical information on the work place exposure, generated by a first database application means from at least three independent files, said files representing the work place data, the product risk data and the personnel data, said reporting system further comprising:
a second database application means for incorporating at least legislative requirement data into said records for each individual; and
a report generating means for generating
a) a schedule for operating the occupational health facility, and/or
b) an epidemiological analysis on the total or part of the individual records.

2. An information reporting system according to claim 1 further comprising a third database application means for incorporating internally set limits on certain exposures or additional exams or also suggested vaccinations into said records for each individual.

3. An information reporting system according to claim 1 further comprising a third database application means for incorporating diagnosis data into the records for each individual.

4. An information reporting system according to claim 2 further comprising a fourth database application means for incorporating diagnosis data into said records for each individual.

5. A method of reporting information about a group of individuals comprising
storing records for the group of individuals, there being at least one record for each individual containing the historical information on the work place exposure of each individual generated from at least three independent files, said files representing the work place data, the product risk data and the personnel data,
storing legislative requirement data,
incorporating at least said legislative requirement data into said records for each individual,
generating a schedule for operating an occupational health facility and/or
generating an epidemiological analysis on the total or part of the individual records.

6. A data processing system for carrying out a method of reporting data comprising
input means for collecting the different data needed,
storage means for at least three independent files representing the work place data, the product risk data and the personnel data,
a first database application means for generating the historical information on the work place exposure of each individual from at least three independent files, said files representing the work place data, the product risk data and the personnel data,
storage means for at least a file representing legislative requirement data,
a second database application means for incorporating at least legislative requirement data into said records for each individual;
a report generating means for generating
a schedule for operating the occupational health facility, and/or
an epidemiological analysis on the total or part of the individual records; and
output means for the presentation of said reports.

7. A method of establishing the work place exposure of an individual using a database application means for generating said work place exposure from at least three independent files, said files representing the work place data, the product risk data and the personnel data.
